(19) Europäisches Patentamt
European Patent Office
Office européen des brevets

(11) Veröffentlichungsnummer: **0 383 285 A2**

# (12) EUROPÄISCHE PATENTANMELDUNG

(21) Anmeldenummer: **90102842.3**

(22) Anmeldetag: **14.02.90**

(51) Int. Cl.[5]: **A61K 31/53, A61K 31/535, A61K 31/54**

(30) Priorität: **16.02.89 DE 3904593**
**11.03.89 DE 3908030**

(43) Veröffentlichungstag der Anmeldung:
**22.08.90 Patentblatt 90/34**

(84) Benannte Vertragsstaaten:
**AT BE CH DE DK FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Mehlhorn, Heinz, Prof. Dr.**
**Steinstrasse 12a**
**D-4040 Neuss-Uedesheim(DE)**
Erfinder: **Taraschewski, Horst, Dr.**
**Dewinkelstrasse 28**
**D-4630 Bochum(DE)**
Erfinder: **Schmahl, Günter, Dr.**
**Marktstrasse 329**
**D-4630 Bochum(DE)**
Erfinder: **Raether, Wolfgang, Dr.**
**Falkensteinstrasse 6**
**D-6072 Dreieich(DE)**
Erfinder: **Rösner, Manfred, Dr.**
**Unter den Buchen 7**
**D-6239 Eppstein/Taunus(DE)**

## (54) Mittel gegen Parasiten bei Fischen und Insekten.

(57) Verbindungen der allgemeinen Formel (I)

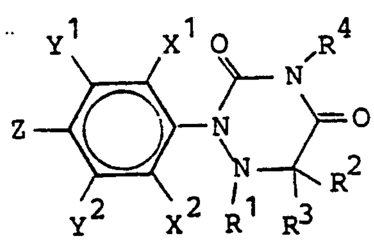

(I)

worin
$R^1$ zusammen mit $R^2$ eine chemische Bindung oder Wasserstoff, Alkyl, Cycloalkyl, Benzyl, das im Phenylring durch Halogen und Alkyl substituiert sein kann, Alkanoyl, das durch Halogen substituiert sein kann, oder Benzoyl, das durch Halogen und Alkyl substituiert sein kann, repräsentiert,
$R^2$ zusammen mit $R^1$ eine chemische Bindung oder Wasserstoff repräsentiert,
$R^3$ Wasserstoff, Alkyl oder Cycloalkyl repräsentiert,
$R^4$ Wasserstoff, Alkyl, Cycloalkyl, Benzyl, das durch Halogen und Alkyl substituiert sein kann, repräsentiert,
$X^1$, $X^2$, $Y^1$, $Y^2$ und Z unabhängig voneinander

a) Wasserstoff, Halogen, Trifluormethyl, Alkyl, Cycloalkyl, Alkoxy, Alkylthio, Alkylsulfinyl, Alkylsulfonyl, Benzylthio, Benzylsulfinyl, Benzylsulfonyl, Nitro, Cyano, Amino, Alkylamino, Dialkylamino, N-Alkylaminoethyl N,N-Dialkylaminomethyl, Piperidino, Morpholino, Thiomorpholino, 1-Pyrrolidinyl, 4-Methyl-1-piperazinyl oder Acylamino oder

b) einen im Phenylring unsubstituierten oder insgesamt 1-, 2- oder 3-fach durch unter a) genannte Reste substituierten Phenoxy-, Phenylthio-, Phenylsulfinyl-, Phenylsulfonyl-, Benzoyl-, Benzoylamino-,

EP 0 383 285 A2

Phenylamino-, N,N-Phenylalkylamino, 1-Cyano-1-phenyl-methyl-, 1-Cyano-1, 1-diphenyl-methyl-, 1-Cyano-1-phenyl-1-($C_1$-$C_6$-alkyl)-methyl-, 1-Cyano-1-phenyl-1-($C_3$-$C_6$-cyclo-alkyl)-methyl-, Thienyl- oder Naphthyl-Rest repräsentieren oder im Falle $R^4$ = H auch ihrer physiologisch verträglichen Salze eignen sich bereits bei geringen Dosierungen zur Bekämpfung eines breiten Spektrums von Endo- und Ektoparasiten bei Fischen oder Insekten.

## Mittel gegen Parasiten bei Fischen und Insekten

Die Erfindung betrifft Mittel gegen parasitäre Protozoen und Metazoen, von denen einige Klassen bei Fischen und Insekten stark verbreitet sind.

Protozoen und Metazoen sind als Parasiten bei Tieren weit verbreitet. Endoparasiten befallen bevorzugt innere Organe und Ektoparasiten Haut oder Augen der Tiere, wodurch erhebliche Schädigungen auftreten können.

Im Falle von Fischen führt ein Parasitieren mancher Protozoen und Metazoen zu Verletzungen von Haut und Kiemen, wodurch die Fische für Infektionen anfällig werden und/oder direkt getötet werden. Andere dieser Parasiten befallen innere Organe der Fische und führen häufig zu Verwachsungen oder zum Tod der Fische. Bei der Massentierhaltung von Fischen in großen Zuchtanlagen können sich parasitäre Protozoen und Metazoen schnell über den ganzen Bestand ausbreiten und bedeuten somit ein großes Risiko für die wirtschaftliche Nutzung dieser Anlagen.

Bei Insekten sind parasitäre Protozoen und Metazoen ebenfalls häufig. Im Falle der Honigbiene sind Protozoen wie Nosema apis weltweit Ursache für schwere Erkrankungen und können neben verminderter Honigproduktion sogar zum Absterben von Bienenvölkern führen. Die Parasiten schädigen die Wirtstiere durch Zerstörung innerer Organe. Dadurch geschwächte Tiere werden häufig anfällig für weitere Erreger. Befall mit Varroamilben schwächt Bienen derartig, daß sie häufig durch die Nosema-Erreger verenden.

Bisher bekannte Mittel zur Bekämpfung von parasitären Protozoen und Metazoen haben meist ein zu enges Wirkungsspektrum. Auch sind manche Mittel zu wenig wirksam, so daß hohe Dosen verabreicht werden müssen, was andererseits die Gefahr von toxischen Effekten erhöht. Aus den genannten Gründen und wegen der Möglichkeit der Resistenzbildung gegen vorhandene Mittel besteht ein ständiger Bedarf nach neuen effektiven Mitteln gegen Parasiten bei Fischen und Insekten.

Gegenstand der Erfindung ist die Verwendung von Verbindungen der allgemeinen Formel (I)

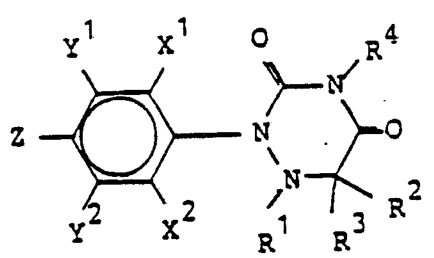

(I)

worin

$R^1$ zusammen mit $R^2$ eine chemische Bindung oder Wasserstoff, geradkettiges oder verzweigtes $C_1$-$C_{12}$-Alkyl, $C_3$-$C_8$-Cycloalkyl, Benzyl, das im Phenylring durch 1 - 3 Reste aus der Gruppe Halogen und $C_1$-$C_4$-Alkyl substituiert sein kann, $C_1$-$C_{12}$-Alkanoyl, das 1- bis 3-fach durch Halogen substituiert sein kann, oder Benzoyl, das durch 1 bis 3 Reste aus der Gruppe Halogen und $C_1$-$C_4$-Alkyl substituiert sein kann, repräsentiert,

$R^2$ zusammen mit $R^1$ eine chemische Bindung oder Wasserstoff repräsentiert,

$R^3$ Wasserstoff, $C_1$-$C_{12}$-Alkyl oder $C_3$-$C_8$-Cycloalkyl repräsentiert,

$R^4$ Wasserstoff, geradkettiges oder verzweigtes $C_1$-$C_{12}$-Alkyl, $C_3$-$C_8$-Cycloalkyl, Benzyl, das durch 1 bis 3 Reste aus der Gruppe Halogen und $C_1$-$C_4$-Alkyl substituiert sein kann, repräsentiert,

$X^1$, $X^2$, $Y^1$, $Y^2$ und Z unabhängig voneinander

a) Wasserstoff, Halogen, Trifluormethyl, $C_1$-$C_6$-Alkyl, $C_3$-$C_6$-Cycloalkyl, $C_1$-$C_6$-Alkoxy, $C_1$-$C_6$-Alkylthio, $C_1$-$C_6$-Alkylsulfinyl, $C_1$-$C_6$-Alkylsulfonyl, Benzylthio, Benzylsulfinyl, Benzylsulfonyl, Nitro, Cyano, Amino, $C_1$-$C_{12}$-Alkylamino, Di-($C_1$-$C_{12}$-alkyl)-amino, N-($C_1$-$C_{12}$-Alkyl)-aminomethyl, N,N-Di-($C_1$-$C_{12}$-alkyl)-amino-methyl, Piperidino, Morpholino, Thiomorpholino, 1-Pyrrolidinyl, 4-Methyl-1-piperazinyl oder $C_1$-$C_6$-Acylamino oder

b) einen im Phenylring unsubstituierten oder insgesamt 1-, 2- oder 3-fach durch unter a) genannte Reste substituierten Phenoxy-, Phenylthio-, Phenylsulfinyl-, Phenylsulfonyl-, Benzoyl-, Benzoylamino-, Phenylamino-, N,N-Phenyl-($C_1$-$C_6$-alkyl)-amino, 1-Cyano-1-phenyl-methyl-, 1-Cyano-1,1-diphenyl-methyl-, 1-Cyano- 1-phenyl- 1-($C_1$-$C_6$-alkyl)-methyl-, 1- Cyano-1-phenyl-1-($C_3$-$C_6$-cycloalkyl)-methyl-, Thienyl- oder Naphthyl-Rest repräsentieren zur Bekämpfung von Endo- und Ektoparasiten bei Fischen und Insekten.

Von besonderem Interesse ist die Verwendung von Verbindungen der allgemeinen Formel (I), in der $X^1$, $X^2$, $Y^1$, $Y^2$ unabhängig voneinander aus der Gruppe der Reste Wasserstoff, Halogen, insbesondere Chlor, Trifluormethyl, $C_1$-$C_4$-Alkyl, $C_1$-$C_4$-Alkoxy, Nitro und Cyano ausgewählt sind.

Bevorzugt werden Verbindungen der allgemeinen Formel (I) verwendet, in der $X^1$ und $X^2$ unabhängig voneinander Wasserstoff oder $C_1$-$C_4$-Alkyl, vorzugsweise Wasserstoff oder Methyl, insbesondere nur Wasserstoff, repräsentieren und $Y^1$ und $Y^2$ unabhängig voneinander aus der Gruppe der Reste Wasserstoff, Halogen, insbesondere Chlor, Trifluormethyl, $C_1$-$C_4$-Alkyl, insbesondere Methyl und Ethyl, ausgewählt sind.

Vorzugsweise werden erfindungsgemäße Verbindungen der allgemeinen Formel (I) eingesetzt, in welcher Z

a) Wasserstoff, $C_1$-$C_4$-Alkoxy, $C_1$-$C_4$-Alkylthio, $C_1$-$C_4$-Alkylsulfinyl, $C_1$-$C_4$-Alkylsulfonyl, Benzylthio, Benzylsulfinyl oder Benzylsulfonyl, oder

b) Phenoxy, Phenylthio, Phenylsulfinyl, Phenylsulfonyl, Benzoyl, Benzoylamino, Phenylamino, 1-Cyano-1-phenyl-methyl, 1-Cyano-1-phenyl-1-($C_1$-$C_6$-alkyl)-methyl, Thienyl oder einen unter b) genannten Rest, der durch 1 bis 3 Reste aus der Gruppe

c) Halogen, insbesondere Chlor, Trifluormethyl, $C_1$-$C_6$-Alkyl, $C_1$-$C_6$-Alkoxy, $C_3$-$C_6$-Cycloalkyl, $C_1$-$C_4$-Alkylthio, $C_1$-$C_4$-Alkylsulfinyl, $C_1$-$C_4$-Alkylsulfonyl, Nitro, Cyano, Amino, $C_1$-$C_4$-Alkylamino, N,N-Di-($C_1$-$C_4$-alkyl)-amino und $C_1$-$C_4$-Acylamino substituiert ist, repräsentiert.

Besonders bevorzugt ist die Verwendung der Verbindungen der allgemeinen Formel (I), in welcher Z Phenoxy, Phenylthio, Phenylsulfinyl, Phenylsulfonyl, 1-Cyano-1-phenyl-methyl oder einen der genannten Reste, der durch 1 oder 2 Substituenten aus der Gruppe Halogen, insbesondere Chlor, Trifluormethyl, $C_1$-$C_4$-Alkyl, $C_1$-$C_4$-Alkoxy, $C_1$-$C_4$-Alkylthio, $C_1$-$C_4$-Alkylsulfinyl und $C_1$-$C_4$-Alkylsulfonyl substituiert ist, repräsentiert.

In den Verbindungen der allgemeinen Formel (I) ist

$R^1$ vorzugsweise zusammen mit $R^2$ eine chemische Bindung oder Wasserstoff, geradkettiges oder verzweigtes $C_1$-$C_4$-Alkyl, insbesondere Methyl oder Ethyl, Benzyl, $C_1$-$C_5$-Alkanoyl oder Benzoyl;

$R^2$ ist vorzugsweise Wasserstoff oder zusammen mit $R^1$ eine chemische Bindung;

$R^3$ ist vorzugsweise Wasserstoff oder $C_1$-$C_4$-Alkyl, insbesondere Methyl oder Ethyl;

$R^4$ ist vorzugsweise Wasserstoff, geradkettiges oder verzweigtes $C_1$-$C_4$-Alkyl, insbesondere Methyl oder Ethyl, oder Benzyl.

Im Falle $R^4$ = H können die Verbindungen der Formel (I) als Salze vorliegen. Die Erfindung betrifft insbesondere auch die physiologisch verträglichen Salze der Verbindung der Formel (I), beispielsweise die Alkali-, Erdalkali- oder gegebenenfalls substituierten Ammoniumsalze.

Besonders bevorzugte Verbindungen der genannten Formel (I) sind solche, die eine Kombination der obengenannten bevorzugten Merkmale enthalten.

Die erfindungsgemäß verwendeten Verbindungen der Formel (I) sind in der Regel bekannt oder können analog bekannten Verfahren hergestellt werden; siehe beispielsweise EP-A-0215 354, EP-A-0154 885, EP-A-0170 316, EP-A-0232 932, DE-A-27 22 537 und DE-A-24 23 972.

Geeignete Verbindungen für die erfindungsgemäße Verwendung sind beispielsweise

2-[3,5-Dichlor-4-(4-methylsulfonyl-phenoxy)-phenyl]-1-methyl-hexahydro-1,2,4-triazin-3,5-dion,
2-[3,5-Dichlor-4-(4-methylthiophenoxy)-phenyl]-1-methyl-hexahydro-1,2,4-triazin-3,5-dion,
2-[3,5-Dichlor-4-(4-methylsulfinylphenoxy)-phenyl]-1-methyl-hexahydro-1,2,4-triazin-3,5-dion,
2-[3,5-Dichlor-4-(3-methyl-4-methylthio)-phenyl]-1-methyl-hexahydro-1,2,4-triazin-3,5-dion,
2-[3,5-Dichlor-4-(4-chlorphenylthio)-phenyl]-1-methyl-hexahydro-1,2,4-triazin-3,5-dion,
2-[3,5-Dichlor-4-(4-methylthio-phenoxy)-phenyl]-hexahydro-1,2,4-triazin-3,5-dion,
2-[3.5-Dichlor-4-(4-methylsulfinyl-phenoxy)-phenyl]-hexahydro-1,2,4-triazin-3,5-dion,
2-[3,5-Dimethyl-4-(4-methylthio-phenoxy)-phenyl]-hexahydro-1,2,4-triazin-3,5-dion,
2-[3,5-Dichlor-4-(3-methyl-4-methylthio-phenoxy)-phenyl]-hexahydro-1,2,4-triazin-3,5-dion,
2-[3,5-Dichlor-4-(4-chlorphenylthio)-phenyl]-hexahydro-1,2,4-triazin-3,5-dion,
2-[3,5-Dichlor-4-(4-methylthio-phenylthio)-phenyl]-hexahydro-1,2,4-triazin-3,5-dion,
2-[3,5-Dichlor-4-(4-methylsulfonyl-phenoxy)-phenyl]-hexahydro-1,2,4-triazin-3,5-dion,
2-[3,5-Dichlor-4-(4-methylsulfonylphenoxy)-phenyl]-1,2,4-triazin-3,5-(2H,4H)-dion,
2-[3,5-Dichlor-4-(4-methylthio-phenoxy)-phenyl]-1,2,4-triazin-3,5-(2H,4H)-dion,
2-[3,5-Dichlor-4-(4-methylsulfinyl-phenoxy)-phenyl]-1,2,4-triazin-3,5- (2H,4H)-dion,
2-[3,5-Dichlor-4-(3-methyl-4-methylthio-phenoxy)-phenyl]-1,2,4-triazin-3,5-(2H,4H)-dion,
2-[3,5-Dichlor-4-(4-chlorphenylthio)-phenyl]-1,2,4-triazin-3,5-(2H,4H)-dion,
2-[3,5-Dichlor-4-(1-cyano-1-(4-chlorphenyl)-methyl)-phenyl]-1,2,4-triazin-3,5-(2H,4H)-dion und
2-[3-Chlor-4-(1-cyano-1-(4-chlorphenyl)-methyl)-phenyl]-1,2,4-triazin-3,5-(2H,4H)-dion.

Die bekannten Verbindungen sind als Mittel zur Bekämpfung von Coccidiose, insbesondere in der

Geflügelhaltung beschrieben (siehe obengenannte Literatur). Es war überraschend, daß sie effektiv gegen Parasiten bei Fischen und Insekten eingesetzt werden können.

Beispiele für Parasiten, die mit den Verbindungen der Formel (I) wirksam bekämpft werden können, sind Fischparasiten aus dem Stamm der Protozoen und Metazoen, insbesondere Protozoen aus der Klasse Ciliata, wie Ichthyophthirius multifiliis, Chilodonella cyprini, Trichodina spp., Glossatella spp., Apiosoma spp., Epistylis spp., oder aus der Klasse Myxozoa, wie Myxobolis cerebralis, Myxosoma spp., Myxidium spp., Myxobolus spp., Henneguya spp. und Hoferellus oder der Klasse der Mikrosporidia, wie Glugea spp., Thelohania spp., und Pleistophora spp., oder Plathelminthen der Klasse Monogenea, wie Dactylogyrus spp., Gyrodactylus spp., Pseudodactylogyrus spp., Diplozoon spp., etc.. Weiterhin kommen als Parasiten bei Fischen Amoeben, Darmflagellaten und Kokzidien in Frage. Beispiele für Parasiten bei Insekten, insbesondere Bienen und Seidenraupen, sind Amoeben, Mikrosporidien, wie Nosema apis bei Bienen, Milben der Acarapis-Arten und Brutmilben der Klasse Varroa.

Bevorzugt ist die Verwendung zur Behandlung von Fischen gegen Parasiten der Haut und der Kiemen.

Die Verbindungen der Formel (I) sind sowohl zur Behandlung von Süß- als auch Salzwasserfischen geeignet. Beispielsweise können Nutz-, Zucht-, Aquarien- und Zierfische unterschiedlichster Altersstufen behandelt werden, insbesondere Nutz- oder Zuchtfische, wie Lachs, Rotauge, Karpfen, Forelle, Aal, Brachse, Weißfisch, Rotfeder, Scholle, Heilbutt, Seezunge, Döbel, Seebrassen (Dicentrarchus spp.), Red seabream (Pagurus major), Grey mullet (Mugil cephalus), Pompano, Gilthread seabream (Sparus auratus), Tilapia spp., Japanaal (Anguilla japonica), Yellow tail (Seriola quinqueradiata) und Chichliden-Arten wie Plagioscion und Channel catfish.

Besonders geeignet ist die erfindungsgemäße Verwendung zur Behandlung von Fischbrut, beispielsweise von Karpfen, Aal und Zierfischen.

Die Behandlung der Fische mit einer Verbindung der Formel (I) kann beispielsweise durch Zusatz der Verbindung oder eines Mittels, das die Verbindung enthält, zu dem Wasser erfolgen, in dem sich die Fische aufhalten. Beispielsweise können die Fische in ein Bad oder Becken eingesetzt und für relativ kurze Zeit, z.B. von Minuten bis zu mehreren Stunden behandelt werden. Eine solche Behandlung ist in Zuchtanlagen gut beim Umsetzen der Fische von einem Zuchtbecken in ein anderes durchzuführen. Alternativ ist eine länger andauernde Behandlung des Lebensraums der Fische, z.B. in Aquarien, Fischbecken, Tanks oder Teichen durchführbar. Ebenso ist es möglich, die Verbindungen der Formel (I) den Fischen oral, beispielsweise über das Futter, zu applizieren.

Die Behandlung der Fische kann auch in Kombination mit anderen Wirkstoffen durchgeführt werden, beispielsweise solchen, die bevorzugt gegen andere Schädlinge wirksam sind.

Zu den Insekten gehören die vom Menschen gehaltenen Nutz-und Zuchtinsekten, wie z.B. Honigbienen, Seidenraupen, Schlupfwespen; ebenso gehören dazu Insekten, die zu Versuchszwecken gezüchtet oder zur Sammlung genetischen Materials gehalten werden. Die Verbindungen der Formel (I) eignen sich zur Behandlung aller Entwicklungsstadien der Insekten.

Eine Behandlung in Kombination mit anderen Wirkstoffen ist möglich, beispielsweise eine Behandlung von Bienen gegen Nosema apis und Varroa jacobsoni mit einer Verbindung der Formel (I) und einem oder mehreren Wirkstoffen aus der Gruppe synthetische Phosphorsäureester wie Coumaphos und Malathion, Pyrethroide wie Flumethrin, Cyfluthrin und Cyalothrin, Amitraz und Cymiazol, Formamidine wie Chlordimeform und Phenothiazine wie Promazin.

Die Behandlung der Insekten kann beispielsweise durch Füttern oder Kontakt der Insekten mit fein verteiltem Wirkstoff, gegebenenfalls in Kombination mit Hilfsstoffen, erfolgen. Der Wirkstoff wird dazu z.B. versprüht, verstäubt, verdampft, verdunstet, verräuchert oder in Trägerstoffen dispergiert oder auf solche aufgebracht.

Die Behandlung kann auch systemisch über die Hämolymphe der Insekten erfolgen, wobei der Wirkstoff dann vorzugsweise dem Futter oder Trinkwasser zugesetzt oder in entsprechender Weise angeboten wird.

Die Behandlung der Insekten kann ganzjährig erfolgen, bei Honigbienen vorzugsweise bei der Wintereinfütterung und/oder in der brutfreien Zeit.

In der Regel werden die wirksamen Verbindungen in bestimmter Weise zubereitet, die der Anwendung angemessen ist. Gegenstand der Erfindung sind deshalb auch Mittel, enthaltend Verbindungen der genannten Formel (I), zur Bekämpfung von Endo- und Ektoparasiten bei Fischen und Insekten.

Geeignete Mittel für die kurzzeitige oder längerfristige Behandlung der Fische in Bädern oder Teichen sind Lösungen der Wirkstoffe in einem oder mehreren polaren Lösungsmitteln, wobei die Lösung beim Verdünnen mit Wasser in der Regel alkalisch reagieren sollen. Derartige Mittel können beispielsweise hergestellt werden, indem der Wirkstoff in einem wasserlöslichen Lösungsmittel gelöst wird, welches entweder selbst alkalisch reagiert oder dem eine wasserlösliche Base zugefügt wird. Die Base kann in dem

Mittel gelöst oder suspendiert vorliegen. Bei Verdünnen des Mittels mit Wasser auf die Anwendungskonzentration soll vorzugsweise ein pH von 7 bis 10, insbesondere von 8 bis 10, eingestellt werden. Für die Herstellung der Mittel in Lösungsform sind wasserlösliche Lösungsmittel geeignet, in denen der Wirkstoff in genügender Konzentration, vorzugsweise 0,4 bis 60 Gew.-%, insbesondere 1 bis 30 Gew.-%, löslich ist, und die physiologisch unbedenklich sind. Geeignete Lösungsmittel sind beispielsweise Alkohole wie Ethanol und Isopropanol, aromatische Alkohole wie Benzylalkohol, Polyhydroxyverbindungen wie Glycerin, Propylenglykol, Polyethylenglykole, Blockpolymere aus Ethylenoxid und Propylenoxid, Aminoalkanole wie Ethanolamin, Diethanolamin, Triethanolamin, Ketone wie Aceton, und Methylethylketon, Ester wie Essigsäureethylester oder Dispergier- und Emulgiermittel, wie Polyethylenglykolether, Polyethylenglykol-alkylamine, Polyethylenglykolstearat, Nonylphenolpolyethylenglykolether, Polyoxyethylensorbitanmonooleat oder polyoxyethyliertes Rizinusöl.

Zur Einstellung des alkalischen Milieus eignen sich Basen wie beispielsweise organische Basen aus der Gruppe der Aminosäuren, speziell L- oder D,L-Arginin und L- oder D,L-Lysin, oder Glucosamin, Methylglucosamin, 2-Amino-2-hydroxymethylpropandiol-(1,3) oder auch N,N,N′,N′-Tetrakis-(2-hydroxypropyl)-ethylendiamin; ebenfalls geeignet sind anorganische Basen, wie Ammoniak oder Natriumcarbonat.

Die erfindungsgemäßen Mittel in Lösungsform können auch weitere Formulierungsstoffe enthalten, vorzugsweise 0,1 bis 20 Gew.-% Hilfsstoffe aus der Gruppe der Antioxidantien, Tenside, Suspensionsstabilisatoren und Verdickungsmittel wie Methylcellulose, Alginate, Polysaccharide, Galactomannane oder kolloidale Kieselsäure. Aufbaustoffe zur Tierernährung, Aroma- und Farbstoffe können ebenfalls zugesetzt werden. Zur Einstellung des pH der Lösung eignen sich vielfach auch Säuren, die mit der enthaltenen Base ein Puffersystem bilden.

Mittel zur oralen Anwendung können Pulver, Granulate, Lösungen, Emulsions- oder Suspensionskonzentrate sein, die in der Regel mit dem Futter homogen vermischt verabreicht werden. Derartige Mittel lassen sich analog üblichen Verfahren herstellen, beispielsweise indem man den Wirkstoff mit festen oder flüssigen Trägerstoffen und eventuell unter Zusatz von Emulgier- oder Dispergiermitteln, Lösungsvermittlern, Farbstoffen, Konservierungsstoffen und/oder Antioxidantien vermischt.

Als feste Trägerstoffe seien z.B. natürliche Gesteinsmehle wie Kaoline, Tonerden, Talkum, Kreide, Diatomeenerde, organische Trägerstoffe wie Rohrzucker, Milchzucker, Traubenzucker, Getreidemehle oder -schrote, Stärke, Tiermehle, Cellulose, Milchpulver, anorganische Trägerstoffe, wie Kochsalz, Carbonate wie Calciumcarbonat oder Hydrogencarbonate, Aluminiumoxide, Kieselsäure und Silikate genannt. Als flüssige Trägerstoffe und Lösungsvermittler sind beispielsweise Wasser, Alkohole wie Ethanol oder Isopropanol, Glykole wie Ethylenglykol, Propylenglykol, Polyethylenglykole, Polypropylenglykole, Blockpolymere von Propylenoxid und Ethylenoxid, Glycerin, aromatische Alkohole wie Benzylalkohol, Phenylethylethanol, und Phenoxyethanol, Ester wie Essigsäureethylester, Essigsäurebutylester und Benzoesäurebenzylester, Alkylenglykolalkylether wie Dipropylenglykolmonomethylether, Diethylenglykolmonobutylether, Ketone wie Aceton und Methylethylketon, aromatische und aliphatische Kohlenwasserstoffe, pflanzliche und synthetische Öle, Dimethylformamid, Dimethylsulfoxid, Dimethylacetamid und N-Methylpyrrolidon geeignet.

Beispiele für geeignete Dispergier- und Emulgiermittel sind nichtionogene Tenside wie polyoxyethyliertes Rizinusöl, Polyoxyethylensorbitanmonoölsäureester, Sorbitanstearinsäureester, Ethanolglycerinmonostearinsäureester, Stearinsäure-polyethylenglykol-ester. Alkylphenolpolyglykolether wie Nonylphenolpolyglykolether, ampholytische Tenside wie Dinatrium-N-lauryl-$\beta$-iminodipropionat oder Lecithin, anionaktive Tenside wie Natriumlaurylsulfat, Fettalkoholethersulfate und kationaktive Tenside wie Cetyltrimethylammoniumchlorid. Die Konzentration des Wirkstoffs kann in Mitteln zur oralen Anwendung in weiten Bereichen variieren und ist vorzugsweise zwischen 0,0001 bis 15 Gew.-%. Erfindungsgemäße Futterzusatzmittel bestehen beispielsweise aus 1 bis 10 Gew.-% der Verbindung der Formel (I) und 49 -90 Gew.-% Sojabohnen-Protein oder aus 0,5 bis 10 Gew.-% Verbindung der Formel (I), 0,05 bis 1,5 Gew.-% Benzylalkohol, bis zu 4 % Hydroxypropylmethylcellulose und Restanteil Wasser.

Die wirksame Dosis an Verbindung der Formel (I) zur Behandlung der Fische hängt von Art und Dauer der Behandlung sowie Alter und Zustand der behandelten Fische ab. Die Dosis ist bei der Behandlung in Bädern in der Regel zwischen 0,1 und 50 mg Wirkstoff pro Liter Wasser. Bei einer kurzen Behandlungsdauer ist die Konzentration vorzugsweise 2 bis 50 mg Wirkstoff pro Liter, insbesondere 5 bis 10 mg Wirkstoff pro Liter Wasser bei einer Behandlungsdauer von 1 bis 5 Stunden. Bei jüngeren Fischen liegt die Konzentration in der Regel niedriger als bei älteren Fischen. Bei längeren Behandlungsdauern kann die Konzentration ebenfalls in der Regel niedriger eingestellt werden. Bei langfristiger Behandlung des Lebensraums der Fische, beispielsweise in Teichen, wird vorzugsweise 0,1 bis 5 mg Wirkstoff pro Liter Wasser verwendet.

Geeignete Mittel zur Sprühbehandlung von Insekten enthalten den Wirkstoff in einer Konzentration 0,1 bis 50 Gew.-%, vorzugsweise 0,3 bis 20 Gew.-% neben Verdünnungsmitteln und/oder Hilfsstoffen, wie

Emulgatoren, die unter den anwendungsgemäßen Konzentrationen insektenverträglich sind. Bei Fütterung soll vorzugsweise eine gesättigte Zuckerlösung als Träger dienen.

Als Verdünnungsmittel kommen beispielsweise Wasser, Alkohole wie Methanol, Ethanol, n- und i-Propanol, Butanol, Pentanol, Hexanol, Heptanol und Octanol, Glycerin, Glykole wie Ethylenglykol, Propylenglykol, 1,3- und 1,4-Butylenglykol sowie entsprechende Glykolmonomethyl- oder -dimethylether, Benzylalkohol und verwandte aromatische Alkohole, Ester wie Essigsäureethylester, -propyl- oder -butylester, Milchsäureethylester, Ketone wie Aceton und Methylethylketon, Mono- und Triglyceride mit natürlichen Fettsäuren, pflanzliche und synthetische Öle, z.B. Ricinusöl, Olivenöl, flüssige aliphatische Kohlenwasserstoffe, Dimethylsulfoxid, Dimethylacetamid, Dimethylformamid und N-Methylpyrrolidon.

Als. Emulgatoren eignen sich anionaktive, kationaktive, nichtionogene und ampholytische Tenside. Beispiele für Emulgatoren sind Fettalkoholsulfate und -ethersulfate, z.B. Natriumlaurylsulfat, Alkylarylsulfonate, Tetraalkylammoniumsalze wie Cetyltrimethylammoniumchlorid, Lecithin, Di-Na-N-lauryl-$\beta$-iminodipropionat, polyoxyethyliertes Ricinusöl, Sorbitanfettsäureester, polyoxyethylierte Sorbitanfettsäureester, Fettsäurepolyglykolether, Alkylphenolpolyglykolether.

Die Mittel zur Sprühbehandlung der Insekten enthalten die Emulgatoren und den Wirkstoff vorzugsweise im Gewichtsverhältnis von 0,05 bis 10:1, insbesondere 0,1 bis 5:1 und werden durch Lösen der Wirkstoffe im Verdünnungsmittel und/oder Emulgator, Zusatz der übrigen Komponenten und Hilfsstoffe und gegebenenfalls weiterem Verdünnen mit Wasser auf die gewünschte Konzentration hergestellt. Die Mittel können in relativ hoher Konzentration nach dem ULV-Verfahren (Ultra-low-volume-Verfahren)mit dafür üblichen Geräten oder mit Hilfe elektrostatischer Aufladung versprüht werden. Konventionell werden die Sprühmittel in der Regel auf Konzentrationen von $10^{-4}$ bis 5 Gew.-% Wirkstoff, vorzugsweise $10^{-3}$ bis 0,5 Gew.-% Wirkstoff mit Wasser verdünnt und mit üblichen Geräten wie Rückenspritze, Kolbenpumpe oder Lackierpistole versprüht.

Geeignete Stäubemittel zur Behandlung von Insekten enthalten neben Wirkstoff insektenverträgliche Trägerstoffe, die zur Herstellung von Pudern und wettable-powder geeignet sind, sowie gegebenenfalls Netzmittel, die den oben angeführten Emulgatoren entsprechen. Anorganische Trägerstoffe sind beispielsweise Talkum, Kaolin, Calciumcarbonat, Silikate, Bentonite; als organische Trägerstoffe seien Stärken wie Reisstärke, Zucker, Cellulose und -derivate genannt. Die Mittel werden durch inniges Vermischen der Komponenten hergestellt.

Andere Mittel und Hilfsmittel zur Behandlung von Insekten, wobei die Wirkstoffe verdampft, verdunstet oder verräuchert werden, können nach für Insektenbehandlungen üblichen Methoden mit Verbindungen der Formel (I) zubereitet und angewendet werden.

Mittel zur Insektenbehandlung, die systemisch wirken, enthalten neben vorzugsweise 0,5 bis 25 %, vorzugsweise 1 bis 10 Gew.-% Wirkstoffen der Formel (I) Futterstoffe wie Zuckerarten in Form von Granulaten, Lösungen, Suspensionen, Emulsionen oder anderen Mischungen. Die Mischungen werden in der Regel mit Wasser oder Zuckerlösung auf Anwendungskonzentrationen von vorzugsweise $10^{-9}$ bis 2 Gew.-%, insbesondere $10^{-4}$ bis 0,1 Gew.-% verdünnt oder stellen feste anwendungsfertige Futterteige oder Knete dar, die den Wirkstoff in der Anwendungskonzentration neben Zucker und Stärke enthalten. Bevorzugt sind systemisch wirkende Mittel, die als wassermischbare Lösungen der Wirkstoffe dem Trinkwasser der Insekten zugesetzt werden können. Diese Mittel enthalten vorzugsweise Hilfsstoffe wie Emulgatoren und Lösungsmittel, wie sie auch für die bereits erwähnten Mittel zur Behandlung von Fischen in Bädern und Teichen geeignet sind, insbesondere die Mittel zur pH-Kontrolle. Die Lösung des Wirkstoffkonzentrats sollte einen pH von 11 vorzugsweise nicht überschreiten. Zur Herstellung der systemisch wirkenden Mittel werden in einfacher Weise die Wirkstoffe mit dem Lösungsmittel und Emulgator so lange vermischt, bis eine klare Lösung entstanden ist.

**Beispiel 1**

In Vivo-Versuche bei Karpfen

Karpfen im Zuchtstadium von 6-8 cm, die stark mit Ektoparasiten aus der Klasse der Monogeneen (25 - 42 Monogeneen der Species Dactylogyrus vastator und Dactylogyrus extensus pro Fisch) bzw. Ciliata (mehrere Hundert Trophozoiten der Species Ichthyophthirius multifiliis pro Fisch) infiziert waren, wurden in Kurzzeitbädern bei 22 °C unter Belüftung mit 2-[3,5-Di-chlor-4-(4-methylsulfonyl-phenoxy)-phenyl]-1-methyl-hexahydro-1,2,4-triazin-3,5-dion bei der angegebenen Konzentration behandelt und nach verschiedenen Inkubationsdauern untersucht. Die Ergebnisse sind in der Tabelle 1 zusammengefaßt.

Unter den verwendeten Konzentrationen treten bei den Fischen keine Unverträglichkeiten gegenüber dem Wirkstoff auf.

Tabelle 1

| Vers.-Nr. | Wirkstoffkonzentration (mg/ml) | Inkubationsdauer | Wirksamkeit bei | |
|---|---|---|---|---|
| | | | D. vastator und D. extensus | I. multifiliis |
| 1 | 1 | 2 h | wenig wirksam, vereinzelt tote Würmer | nicht wirksam |
| 2 | 1 | 3 h | wenig wirksam, vereinzelt tote Würmer | nicht wirksam |
| 3 | 5 | 1 h | 30 % tote Würmer,<br>30 % mit verminderter Beweglichkeit,<br>40 % normal | nicht wirksam |
| 4 | 5 | 2 h | 90 % tote Würmer,<br>10 % verminderte Beweglichkeit; | nicht wirksam |
| 5 | 5 | 3 h | 10 % verminderte Beweglichkeit; | nicht wirksam |
| 6 | 10 | 1 h | 100 % tote Würmer | nicht wirksam |
| 7 | 10 | 2 h | 100 % tote Würmer | - vereinzelt tote Trophozoiten (Bodensatz) |
| 8 | 10 | 3 h | 100 % tote Würmer | - etwas mehr Wirkung als in Versuch 7 |
| 9 | 10 | 4 h | 100 % tote Würmer | - Fische symptomfrei |
| 10 | 15 | 1 h | 100 % tote Würmer | - nicht wirksam |
| 11 | 15 | 2 h | 100 % tote Würmer | - vereinzelt tote Tropozoiten |
| 12 | 15 | 3 h | 100 % tote Würmer | - 80 % tote Trophozoiten |

Beispiel 2

In vitro-Inkubation von Trophozoiten (Ichthyophthirius multifiliis) aus Karpfenkiemen oder Aalkiemen

In Wasser gehaltene Trophozoiten der Größe 150 bis 900 µm wurden auf eine Konzentration von 50 Trophozoiten/100 ml Wasser eingestellt und in Boverischalen mit 2-[3,5-Dichlor-4-(4-methylsulfonyl-phenoxy)-phenyl]-1-methyl-hexahydro-1,2,4-triazin-3,5-dion in der angegebenen Konzentration versetzt; Temperatur 20 °C. Die Ergebnisse sind in Tabelle 2 zusammengefaßt.

Tabelle 2

| Vers.-Nr. | Wirkstoffkonzentration [mg/ml] | Inkubationsdauer | Wirkung |
|---|---|---|---|
| 1 | 5 | 1 h | 100 % Cytolyse |
| 2 | 10 | 1 h | 100 % Cytolyse |
| 3 | 15 | 1 h | 100 % Cytolyse |
| 4 | 10 | 15 min | 100 % Cytolyse |
| 5 (Kontrolle) | 0 | 12 | keine Cytolyse; Tomitenbildung |

Beispiel 3

In vivo-Versuche bei Zierfischen

Jeweils 10 Zierfische der Species Neon rot (paracheirodon axelrodi, 18 - 25 mm), Schwertträger (Xiphophorus helleri) und Zebrabärblinge (Brachydanio rerio, 30 - 32 mm) wurden in Kurzzeitbädern bei 25 °C unter Belüftung mit 10 mg/l 2-[3,5-Dichlor-4-(4-methylsulfonyl-phenoxy)-phenyl]-1-methyl-hexahydro-1,2,4-triazin-3,5-dion, das dem Badwasser direkt zugesetzt wurde, behandelt und somit die Verträglichkeit getestet. Unter analogen Bedingungen wurden mit Ichthyophthirius multifiliis infizierte Fische behandelt. Die Ergebnisse sind in der Tabelle 3 zusammengestellt.

Tabelle 3

| Vers.-Nr. | Fischspecies | infiziert | Behandlungsdauer | Parasiten pro Fisch | Effekt auf Parasiten | Verträglichkeit |
|---|---|---|---|---|---|---|
| 1 | Neon rot | nein | 4 h | - | - | 100 % |
| 2 | Neon rot | ja | 4 h | vereinzelte Trophozoiten noch auf Fischen | viele tote Trophozoiten am Boden | 100 % |
| 3 | Schwertträger | nein | 4 h | - | - | 100 % |
| 4 | Schwertträger | ja | 4 h | vereinzelte Trophozoiten noch auf Fischen | viele tote Trophozoiten am Boden; Fische symptomfrei nach 4 Tagen | 100 % |
| 5 | Zebrabärblinge | nein | 4 h | - | - | 100 % |
| 6 | Zebrabärblinge | ja | 4 h | vereinzelte Trophozoiten auf Fischen | viele Trophozoiten am Boden | 100 % |

Beispiel 4

In vivo-Behandlung von Stichlingen

Die Wirksamkeit von 10 mg/l 2-[3,5-Dichlor-4-(4-methyl-sulfonyl-phenoxy)-phenyl]-1-methyl-hexahydro-1 ,2,4-triazin-3,5-dion gegen Parasiten wurde an einer größeren Zahl von infizierten Stichlingen untersucht, die mit den in der Tabelle 4 angegebenen Monogeneen-, Ciliaten- bzw. Mikrosporidia-Spezies infiziert waren (siehe Tabelle 4).

Tabelle 4

| Vers.-Nr. | Infektion durch | Inkubationsdauer | Effekte auf Fisch | Effekte auf Parasiten |
|---|---|---|---|---|
| 1 | Gyrodactylus arcuatus | 1 h | keine Würmer auf Fisch | Tote Würmer im Bodensatz |
| 2 | Gyrodactylus arcuatus | 4 h | keine Würmer auf Fisch | Lysierte Würmer nicht mehr nachweisbar |
| 3 | Trichodina spp. | 4 h | Fische 100 % parasitenfrei | Alle Parasiten abgetötet |
| 4 | Glugea anomala | 4 h | Fische 100 % parasitenfrei | Meronten und Sporoblasten völlig zerstört |

Beispiel 5

In vitro-Behandlung von Monogeneen

In Wasser gehaltene Monogeneen der Spezies Pseudodactylogyrus bini wurde analog Beispiel 2 bei einer Konzentration von 10 mg/l desselben Wirkstoffes behandelt. Nach 2 1/4 Stunden waren bereits 90 % der Würmer abgetötet.

Beispiel 6

Analog Beispiel 1 wurde die Wirksamkeit von 2-[3,5-Dichlor-4-(1-cyano-1-(4-chlorphenyl)-methyl)-phenyl]-1,2,4-triazin-3,5-(2H,4H)-dion gegen Protozoa (Trichodina sp.; Apiosoma sp.) und Monogenea (Gyrodactylus arcuatus) an Karpfen und Stichlingen getestet (s. Tabelle 5)

Tabelle 5

| Vers.-Nr. | Infektion durch | Konzentration (mg/l/Dauer (h) | Effekte auf Parasiten an Karpfen und Stichlingen |
|---|---|---|---|
| 1 | Trichodina sp. | 10/1 | 100 % letal |
| 2 | Apiosoma sp. | 10/1 | 100 % letal |
| 3 | Gyrodactylus arcuatus | 5/1 | 100 % letal |

Beispiel 7

Beispiel 6 wurde wiederholt, wobei jedoch als Wirkstoff 2-[3-Chlor-4-(1-cyano-1-(4-chlorphenyl)-methyl)-phenyl]-1,2,4-triazin-3,5-(2H,4H)-dion eingesetzt wurde. Es wurden entsprechend gute Ergebnisse erhalten.

**Ansprüche**

1. Verwendung von Verbindungen der allgemeinen Formel (I)

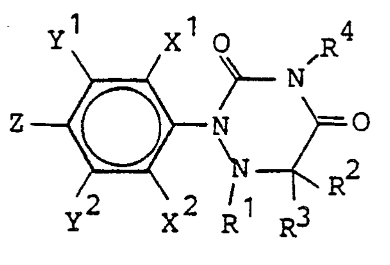

(I)

worin

$R^1$ zusammen mit $R^2$ eine chemische Bindung oder Wasserstoff, geradkettiges oder verzweigtes $C_1$-$C_{12}$-Alkyl, $C_3$-$C_8$-Cycloalkyl, Benzyl, das im Phenylring durch 1 - 3 Reste aus der Gruppe Halogen und $C_1$-$C_4$-Alkyl substituiert sein kann, $C_1$-$C_{12}$-Alkanoyl, das 1- bis 3-fach durch Halogen substituiert sein kann, oder Benzoyl, das durch 1 bis 3 Reste aus der Gruppe Halogen und $C_1$-$C_4$-Alkyl substituiert sein kann, repräsentiert,

$R^2$ zusammen mit $R^1$ eine chemische Bindung oder Wasserstoff repräsentiert,

$R^3$ Wasserstoff, $C_1$-$C_{12}$-Alkyl oder $C_3$-$C_8$-Cycloalkyl repräsentiert,

$R^4$ Wasserstoff, geradkettiges oder verzweigtes $C_1$-$C_{12}$- Alkyl, $C_3$-$C_8$-Cycloalkyl, Benzyl, das durch 1 bis 3 Reste aus der Gruppe Halogen und $C_1$-$C_4$-Alkyl substituiert sein kann, repräsentiert,

$X^1$, $X^2$, $Y^1$, $Y^2$ und Z unabhängig voneinander

a) Wasserstoff, Halogen, Trifluormethyl, $C_1$-$C_6$-Alkyl, $C_3$-$C_6$-Cycloalkyl, $C_1$-$C_6$-Alkoxy, $C_1$-$C_6$-Alkylthio, $C_1$-$C_6$-Alkylsulfinyl, $C_1$-$C_6$-Alkylsulfonyl, Benzylthio, Benzylsulfinyl,( Benzylsulfonyl, Nitro, Cyano, Amino, $C_1$-$C_{12}$-Alkylamino, Di-($C_1$-$C_{12}$-alkyl)-amino, N-($C_1$-$C_{12}$-Alkyl)- aminomethyl, N,N-Di-($C_1$-$C_{12}$-alkyl)-amino-methyl, Piperidino, Morpholino, Thiomorpholino, 1-Pyrrolidinyl, 4-Methyl-1-piperazinyl oder $C_1$-$C_6$-Acylamino oder

b) einen im Phenylring unsubstituierten oder insgesamt 1-, 2- oder 3-fach durch unter a) genannte Reste substituierten Phenoxy-, Phenylthio-, Phenylsulfinyl-, Phenylsulfonyl-, Benzoyl-, Benzoylamino-, Phenylamino-, N,N-Phenyl-($C_1$-$C_6$-alkyl)-amino, 1-Cyano-1-phenyl-methyl-, 1-Cyano- 1,1-diphenyl-methyl-, 1-Cyano-1-phenyl-1-($C_1$-$C_6$-alkyl)-methyl-, 1-Cyano-1-phenyl-1-($C_3$-$C_6$-cycloalkyl)-methyl-, Thienyl- oder Naphthyl-Rest repräsentieren

oder im Falle $R^4$ = H auch ihrer physiologisch verträglichen Salze zur Bekämpfung von Endo- und Ektoparasiten bei Fischen oder Insekten.

2. Verwendung nach Anspruch 1, dadurch gekennzeichnet, daß $X^1$, $X^2$, $Y^1$, $Y^2$ unabhängig voneinander aus der Gruppe der Reste Wasserstoff, Halogen, insbesondere Chlor, Trifluormethyl, $C_1$-$C_4$-Alkyl, $C_1$-$C_4$-Alkoxy, Nitro und Cyano ausgewählt sind.

3. Verwendung nach Anspruch 1 oder 2, dadurch gekennzeichnet, daß $X^1$ und $X^2$ unabhängig voneinander Wasserstoff oder $C_1$-$C_4$-Alkyl, vorzugsweise Wasserstoff oder Methyl, insbesondere nur Wasserstoff, repräsentieren und $Y^1$ und $Y^2$ unabhängig voneinander aus der Gruppe der Reste Wasserstoff, Halogen, insbesondere Chlor, Trifluormethyl, $C_1$-$C_4$-Alkyl, insbesondere Methyl und Ethyl, ausgewählt sind.

4. Verwendung nach einem oder mehreren der Ansprüche 1 bis 3, dadurch gekennzeichnet, daß Z

a) Wasserstoff, $C_1$-$C_4$-Alkoxy, $C_1$-$C_4$-Alkylthio, $C_1$-$C_4$-Alkylsulfinyl, $C_1$-$C_4$-Alkylsulfonyl, Benzylthio, Benzylsulfinyl oder Benzylsulfonyl, oder

b) Phenoxy, Phenylthio, Phenylsulfinyl, Phenylsulfonyl, Benzoyl, Benzoylamino, Phenylamino, 1-Cyano-1-phenyl-methyl, 1-Cyano-1-phenyl-1-($C_1$-$C_6$-alkyl)-methyl, Thienyl oder einen unter b) genannten Rest, der durch 1 bis 3 Reste aus der Gruppe

c) Halogen, insbesondere Chlor, Trifluormethyl, $C_1$-$C_6$-Alkyl, $C_1$-$C_6$-Alkoxy, $C_3$-$C_6$-Cycloalkyl, $C_1$-$C_4$-Alkylthio, $C_1$-$C_4$-Alkylsulfinyl, $C_1$-$C_4$-Alkylsulfonyl, Nitro, Cyano, Amino, $C_1$-$C_4$-Alkylamino, N,N-Di-($C_1$-$C_4$-alkyl)-amino und $C_1$-$C_4$-Acylamino substituiert ist, repräsentiert.

5. Verwendung nach einem oder mehreren der Ansprüche 1 bis 4, dadurch gekennzeichnet, daß Z Phenoxy, Phenylthio, Phenylsulfinyl, Phenylsulfonyl, 1-Cyano-1-phenyl-methyl oder einen der genannten Reste, der durch 1 oder 2 Substituenten aus der Gruppe Halogen, insbesondere Chlor, Trifluormethyl, $C_1$-$C_4$-Alkyl, $C_1$-$C_4$-Alkoxy, $C_1$-$C_4$-Alkylthio, $C_1$-$C_4$-Alkylsulfinyl und $C_1$-$C_4$-Alkylsulfonyl substituiert ist, repräsentiert.

6. Verwendung nach einem oder mehreren der Ansprüche 1 bis 5, dadurch gekennzeichnet, daß $R^1$ zusammen mit $R^2$ eine chemische Bindung oder alleine Wasserstoff, geradkettiges oder verzweigtes $C_1$-$C_4$-Alkyl, insbesondere Methyl oder Ethyl, Benzyl, $C_1$-$C_5$-Alkanoyl oder Benzoyl,

$R^3$ Wasserstoff oder $C_1$-$C_4$-Alkyl, insbesondere Methyl oder Ethyl und

$R^4$ Wasserstoff, geradkettiges oder verzweigtes $C_1$-$C_4$-Alkyl, insbesondere Methyl oder Ethyl, oder Benzyl bedeuten.

7. Verwendung nach einem oder mehreren der Ansprüche 1 bis 6 zur Bekämpfung von Fischparasiten, dadurch gekennzeichnet, daß das Wasser, in dem sich die Fische befinden, 0,1 bis 50 mg Verbindung der Formel (I) pro Liter Wasser enthält.

8. Mittel zur Bekämpfung von Endo- oder Ektoparasiten bei Fischen, dadurch gekennzeichnet, daß es ein oder mehrere Verbindungen der Formel (I) nach einem oder mehreren der Ansprüche 1 bis 6 und ein oder mehrere für die Fischbehandlung übliche Hilfsmittel enthält.

9. Mittel nach Anspruch 8, dadurch gekennzeichnet, daß es ein Futterzusatzmittel ist.

10. Mittel nach Anspruch 8, dadurch gekennzeichnet, daß es eine Lösung des Wirkstoffs in einem wassermischbaren organischen oder wäßrig-organischen Lösungsmittel und gegebenenfalls übliche Formulierungsstoffe aus der Gruppe der Antioxidantien, Tenside, Suspensionsstabilisatoren, Verdickungsmittel, Aufbaustoffe zur Fischernährung, Aroma-und Farbstoffe sowie eine Base enthält und bei Verdünnen mit Wasser alkalisch reagiert.

11. Mittel zur Bekämpfung von Parasiten bei Insekten, dadurch gekennzeichnet, daß es ein oder mehrere Verbindungen der Formel (I) nach einem oder mehreren der Ansprüche 1 bis 6 und ein oder mehrere für die Insektenbehandlung übliche Hilfsmittel enthält.

12. Mittel nach Anspruch 11, dadurch gekennzeichnet, daß es ein Sprüh- oder Stäubemittel ist.

13. Mittel nach Anspruch 11, dadurch gekennzeichnet, daß es ein Futtermittel ist.

14. Mittel nach Anspruch 13, dadurch gekennzeichnet, daß es ein Mittel zur Anwendung über das Trinkwasser ist.

15. Verfahren zur Bekämpfung von Endo- oder Ektoparasiten bei Fischen oder Insekten, dadurch gekennzeichnet, daß man diese oder deren Lebensraum mit einer oder mehreren Verbindungen der Formel I gemäß einem oder mehreren der Ansprüche 1 - 6 behandelt.

16. Verfahren zur Bekämpfung von Endo- oder Ektoparasiten bei Fischen, dadurch gekennzeichnet, daß man diese oder deren Lebensraum mit einem Mittel gemäß einem oder mehreren der Ansprüche 8 - 10 behandelt.

17. Verfahren zur Bekämpfung von Parasiten bei Insekten, dadurch gekennzeichnet, daß man diese oder deren Lebensraum mit einem Mittel gemäß einem oder mehreren der Ansprüche 11-14 behandelt.